# FASCICULE DE BREVET EUROPEEN

(11) **EP 0 752 271 B1**
(45) Date de publication et mention de la délivrance du brevet: **20.09.2000**
(21) Numéro de dépôt: 96401269.4
(22) Date de dépôt: 12.06.1996
(51) Int. Cl.: B01J 20/08, C07C 7/12, C08F 6/02

(54) **Procédé pour l'adsorption de composés organométalliques chélatés**
Verfahren zur Adsorption von chelierten organometallischen Verbindungen
Process for adsorption of chelated organometallic compounds

(30) Priorité: 07.07.1995 FR 9508227
(43) Date de publication de la demande: 08.01.1997
(73) Titulaire: INSTITUT FRANCAIS DU PETROLE, 92852 Rueil-Malmaison Cedex (FR)
(72) Inventeur: Le Loarer, Jean-Luc, 30340 Salindres (FR); Nedez, Christophe, 92600 Asnieres sur Seine (FR)

(56) Documents cités:
- EP-A- 0 379 394
- EP-A- 0 603 990
- WO-A-90/04007
- DE-A- 3 029 802
- FR-E- 73 525
- US-A- 3 067 128

## Description

L'invention concerne un procédé pour l'adsorption de composés organométalliques chélatés d'adsorbants à base d'alumine dans lequel on met en contact lesdits composés avec un adsorbant à base d'alumine présentant des caractéristiques adaptées. Elle concerne plus particulièrement un procédé pour la purification des polyoléfines obtenues par polymérisation d'oléfines en présence de catalyseurs de coordination.

Les polyoléfines sont préparées généralement par polymérisation de monomères avec ajout possible de comonomères tels que le 1-butène, le 1-octène, ... en présence de catalyseurs de polymérisation comprenant des éléments des groupes IVB, VB, VIB du classement périodique des éléments et plus particulièrement le vanadium, le titane, le zirconium. Ces catalyseurs comprennent également comme agents réducteurs des composés organométalliques (alkyl métallique), hydrures métalliques ou hydroxydes de métaux. Ces catalyseurs, généralement appelés catalyseurs de transition, présentent une grande activité catalytique pour la polymérisation d'oléfines.

Cependant, une fois la polymérisation achevée, les poyoléfines obtenues se trouvent polluées par les résidus métalliques provenant des catalyseurs et il est donc indispensable de les épurer avant leur utilisation, pour éviter toute toxicité comme une coloration ou une dégradation parasites.

En outre, les procédés de polymérisation des oléfines comprennent généralement une étape de récupération des monomères n'ayant pas réagi au cours de la polymérisation et des solvants contenus dans les polyoléfines, ces solvants et monomères étant recyclés dans l'unité de polymérisation. La présence des métaux dans ces composés engendre des problèmes de corrosion de l'installation.

Pour éliminer les résidus métalliques issus des catalyseurs, un procédé consiste à mettre en contact le milieu issu de la polymérisation avec des composés organiques. De ce fait, il se crée une réaction de complexation entre les résidus métalliques et les composés organiques introduits, conduisant à des composés organométalliques chélatés.

Ensuite, afin de séparer ces composés organométalliques chélatés des polyoléfines, il est connu d'utiliser différents adsorbants, notamment des alumines (cf. FR-A-1 177 876).

Parmi les adsorbants utilisés, il est connu d'utiliser des adsorbants sous forme de billes. Ces billes sont mises en forme par technologie tournante type drageoir ou tambour tournant (cf EP-A-0 737 512).

Un but de la présente invention est de proposer des adsorbants à base d'alumine pour l'adsorption des composés organométalliques chélatés présentant un taux d'adsorption amélioré par rapport à ceux des produits de l'art antérieur et particulièrement par rapport aux billes d'alumine issues d'une mise en forme par technologie tournante.

Dans ce but, l'invention concerne un procédé pour radsorption de composés organométalliques chélatés dans lequel on met en contact lesdits composés avec un adsorbant à base d'alumine issu d'une mise en forme par coagulation en gouttes ou par extrusion ou obtenu par concassage et présentant un volume des pores de diamètre supérieur à 80 Å d'au moins 0,15 cm³/g et une granulométrie inférieure à 4 mm.

L'invention concerne en outre des adsorbants à base d'alumine comprenant un composé organométallique chélaté obtenus après la mise en oeuvre du procédé défini ci-dessus.

L'invention concerne tout d'abord un procédé pour l'adsorption de composés organométalliques chélatés dans lequel on met en contact lesdits composés avec un adsorbant à base d'alumine issu d'une mise en forme par coagulation en gouttes ou par extrusion ou obtenu par concassage et présentant un volume des pores de diamètre supérieur à 80 Å d'au moins 0,15 cm³/g et une granulométrie inférieure à 4 mm.

Le procédé selon l'invention peut mettre en oeuvre des adsorbants pouvant se présenter sous diverses formes.

Il peut s'agir tout d'abord de billes d'alumine issues d'une mise en forme par coagulation en gouttes. Ce type de billes peut par exemple être préparé par un procédé selon l'enseignement des brevets EP-B-0 015 801 ou EP-B-0 097 539. Le contrôle de la porosité peut être réalisé en particulier selon le procédé décrit dans le brevet EP-B-0 097 539 par coagulation en gouttes d'une suspension ou d'une dispersion aqueuse d'alumine ou d'une solution d'un sel basique d'aluminium se présentant sous forme d'une émulsion constituée d'une phase organique, d'une phase aqueuse et d'un agent de surface ou d'un émulsionnant. Ladite phase organique peut en particulier être un hydrocarbure, l'agent surfactant ou émulsionnant est par exemple du Galoryl EM 10®.

Il peut également s'agir de concassés d'alumine. Ces concassés peuvent être issus du concassage de tout type de matière à base d'alumine telle que, par exemple, des billes obtenues par tous types de procédé (coagulation en gouttes, drageoir ou tambour tournant), des extrudés. Le contrôle de la porosité de ces concassés se fait par le choix de la matière à base d'alumine que l'on concasse pour les obtenir.

II peut enfin s'agir d'extrudés alumine. Ceux-ci peuvent être obtenus par malaxage puis extrusion d'une matière à base d'alumine, ladite matière pouvant être issue de la déshydratation rapide d'hydrargillite (alumine flash) ou de la précipitation d'un gel d'alumine. Le contrôle de la porosité des extrudés peut être réalisé par le choix de l'alumine mise en oeuvre et par les conditions de préparation de cette alumine ou par les conditions opératoires de malaxage de cette alumine avant extrusion. L'alumine peut ainsi être mélangée lors du malaxage à des porogènes. A titre d'exemple, les extrudés peuvent être préparés par le procédé de préparation décrit dans le brevet US 3.856.708.

Le procédé selon l'invention met en général en oeuvre des adsorbants présentant un volume de pores de diamètre supérieur à 80 Å supérieur à 0,15 cm³/g, de préférence supérieur à 0,3 et encore plus préférentiellement supérieur à 0,4 cm³/g.

Le volume des pores de diamètre supérieur à 80 Å représente le volume cumulé créé par tous les pores de taille supérieure à un diamètre de 80 Å. Ce volume est mesuré par la technique de la pénétration du mercure, dans laquelle on applique la loi de Kelvin.

La granulométrie correspond dans le cas d'une mise en forme par coagulation en gouttes au diamètre des billes, dans le cas d'extrudés au diamètre de leur section transversale et dans le cas de concassés à la longueur de leur plus grande section. En général, on met en oeuvre des adsorbants de granulométrie inférieure à 4 mm. Dans le cas des billes mises en forme par coagulation en gouttes ou des extrudés, on peut avantageusement utiliser des adsorbants de granulométrie inférieure à 3 mm, encore plus avantageusement inférieure à 2,4 mm.

De manière préférentielle, le procédé selon l'invention met en oeuvre des adsorbants comprenant au moins un composé d'un élément choisi dans le groupe comprenant les alcalins, les alcalino-terreux.

Ce composé de l'élément peut être un oxyde, un hydroxyde, un sel de l'élément ou un mélange de ceux-ci. On peut citer, à titre d'exemple, en plus des hydroxydes, les sulfates, nitrates, halogénures, acétates, formiates, carbonates et plus généralement les sels d'acides carboxyliques par exemple.

On utilise de préférence les éléments choisis parmi le sodium, le potassium et le calcium.

La teneur du composé de l'élément choisi dans le groupe des alcalins, des alcalino-terreux peut être comprise entre 15 mmole et 750 mmole pour 100 g d'alumine, de préférence entre 15 et 500 mmole pour 100 g d'alumine, encore plus préférentiellement comprise entre 15 et 150 mmole.

L'incorporation de ces éléments peut être effectuée selon l'enseignement de la demande de brevet EP-A-0 379 394.

Selon un premier mode préférentiel de mise en oeuvre du procédé selon l'invention, on utilise des concassés présentant une surface spécifique supérieure à 200 m²/g. Cette surface spécifique est une surface BET. On entend par surface BET, la surface spécifique déterminée par adsorption d'azote conformément à la norme ASTM D 3663-78 établie à partir de la méthode BRUNAUER - EMMETT - TELLER décrite dans le périodique "The Journal of the american Society", 60, 309 (1938).

Dans le cas où le procédé met en oeuvre des adsorbants obtenus par concassage, on utilise de préférence ceux présentant une surface spécifique supérieure à 200 m²/g.

Lorsque le procédé selon l'invention met en oeuvre des billes ou des extrudés, il est avantageux que ces derniers présentent une surface spécifique d'au moins 20 m²/g.

Le procédé selon l'invention permet d'obtenir des taux d'adsorption des composés organométalliques chélatés améliorés, qui peuvent atteindre plus de 75%, ce dernier taux représentant le taux de métal adsorbé par les billes par rapport à la quantité de départ de métal introduite dans le milieu réactionnel et dans les conditions définies dans le test d'adsorption ci-après.

La présente invention concerne plus particulièrement le procédé mettant en oeuvre les adsorbants décrits ci-dessus pour l'adsorption de tout composé organométallique chélaté, plus particulièrement à base des métaux choisis parmi ceux des groupes IVB, VB, VIB, VIIB, VIII, IB, IIB et encore plus particulièrement ceux à base de vanadium, de titane, de zirconium ou de cuivre.

Le procédé selon l'invention convient particulièrement pour l'adsorption de tout composé organométallique qui a été chélaté par des composé organiques tels que l'acétylacétone, le 2-éthylhexanediol-1,3, le di-2-éthyl-hexylphosphate.

De ce fait, le procédé selon l'invention peut convenir pour la purification des polyoléfines obtenues par polymérisation d'oléfines en présence d'un système de catalyseur de coordination. Le procédé de purification peut être du type de celui qui a été décrit plus haut dans l'introduction de la présente description et dans lequel les adsorbants sont mis en contact avec le milieu issu de la polymérisation lui-même préalablement mis en présence de composés organiques.

Dans le procédé selon l'invention, lesdits adsorbants à base d'alumine sont mis en contact avec les composés organométalliques et les adsorbent. A l'issue du procédé d'adsorption des composés organométalliques, les adsorbants sont retirés du réacteur et on obtient des adsorbants à base d'alumine issus d'une mise en forme par coagulation en gouttes ou par extrusion ou obtenus par concassage et présentant un volume des pores de diamètre supérieur à 80 Å d'au moins 0,15 cm³/g et une granulométrie inférieure à 4 mm sur lesquels sont adsorbés les composés organométalliques.

Ces adsorbants peuvent être directement utilisés en tant que catalyseurs métalliques supportés dans tout type de catalyse par métaux précieux adaptée à la nature du métal adsorbé.

Les exemples suivants illustrent l'invention, sans en limiter, toutefois, sa portée.

### EXEMPLES

### Test d'adsorption

Les tests d'adsorption ont été conduits sur des adsorbants activés au préalable à 300°C durant 2 h afin d'éliminer toute trace d'humidité suite à leur stockage et afin de pouvoir comparer leur efficacité dans des conditions identiques.

Les adsorbants sont introduits dans un bêcher contenant du vanadium chélaté par l'acétylacétonate (VO(acac)₂) présent dans 200 ml de toluène à la concentration de 0,1% (en poids par rapport au volume de toluène). Ils sont laissés, sous agitation, au contact du composé durant 48 h à 25°C à l'abri de l'air. Le taux d'adsorption du vanadium chélaté par l'acétylacétonate par l'alumine est mesuré par évolution de la concentration de la solution mesurée par spectroscopie UV-visible.

### EXEMPLE 1 - Concassés d'alumine.

Les adsorbants 1 à 5 sont obtenus par concassage de billes d'alumine obtenues par mise en forme d'alumine issue de la déshydratation rapide d'hydrargillite par technologie tournante.

| | Type | V80Å (cm³/g) | Granulométrie (mm) | Surface spécifique (cm²/g) | Taux d'adsorption |
|---|---|---|---|---|---|
| Adsorbant 1 | concassés | 0,25 | 1,3 | 343 | 91 |
| Adsorbant 2 | concassés | 0,25 | 2,1 | 343 | 85,5 |
| Adsorbant 3 | concassés | 0,25 | 2,8 | 343 | 79 |
| Adsorbant 4 comparatif | concassés | 0,08 | 1,7 | 254 | 68 |
| Adsorbant 5 comparatif | concassés | 0,25 | 4,5 | 343 | 63,5 |

On constate que les concassés d'alumine présentant un volume de pores de diamètre supérieur à 80 Å supérieur à 0,2 cm³/g, et une granulométrie inférieure à 4 mm ont un taux d'adsorption élevé.

### EXEMPLE 2 - Billes d'alumine mises en forme par coagulation en gouttes et extrudés.

Les adsorbants testés sont mis en forme soit par coagulation en gouttes à partir d'une alumine issue de la précipitation d'un gel d'alumine (adsorbant 8), soit par extrusion de ce gel d'alumine (adsorbants 6 et 7).

| | Type | V80Å (cm³/g) | Granulométrie (mm) | Surface spécifique (cm²/g) | Taux d'adsorption |
|---|---|---|---|---|---|
| Adsorbant 6 | extrudés | 0,55 | 1,2 | 211 | 89 |
| Adsorbant 7 | extrudés | 0,55 | 1,6 | 212 | 80 |
| Adsorbant 8 | coagulation en gouttes | 0,54 | 2,0 | 191 | 81,5 |

On constate que ces adsorbants présentant un volume des pores de diamètre supérieur à 80 Å supérieur à 0,2 cm³/g et une granulométrie inférieure à 4 mm présentent un taux d'adsorption élevé.

### EXEMPLE COMPARATIF 3 - Billes d'alumine issues d'une mise en forme au drageoir tournant.

| | Type | V80Å (cm³/g) | Granulométrie (mm) | Surface spécifique (cm²/g) | Taux d'adsorption |
|---|---|---|---|---|---|
| Adsorbant 11 comparatif | drageoir | 0,15 | 1,6 | 356 | 59 |
| Adsorbant 12 comparatif | drageoir | 0,55 | 3,2 | 100 | 68,5 |

On constate que des billes d'alumine obtenues par mise en forme d'alumine issue de la déshydratation rapide d'hydrargillite au drageoir tournant présentent un taux d'adsorption inférieur à celui des adsorbants selon l'invention.

## Revendications

1. Procédé pour l'adsorption de composés organométalliques chélatés caractérisé en ce qu'on met en contact lesdits composés avec un adsorbant à base d'alumine issu d'une mise en forme par coagulation en gouttes ou par extrusion ou obtenu par concassage et présentant un volume des pores de diamètre supérieur à 80 Å d'au moins 0,15 cm³/g et une granulométrie inférieure à 4 mm.

2. Procédé selon la revendication 1 caractérisé en ce que l'adsorbant comprend au moins un composé d'un élément choisi dans le groupe comprenant les alcalins, les alcalino-terreux.

3. Procédé selon la revendication 2 caractérisé en ce que la teneur du composé de l'élément choisi dans le groupe des alcalins, des alcalino-terreux est comprise entre 15 mmole et 750 mmole pour 100 g d'alumine.

4. Procédé selon l'une des revendications 1 à 3 caractérisé en ce que l'adsorbant à base d'alumine est obtenu par concassage et présente une surface spécifique supérieure à 200 m²/g.

5. Procédé selon l'une des revendications 1 à 4 caractérisé en ce que l'adsorbant à base d'alumine est obtenu par concassage de billes d'alumine.

6. Procédé selon l'une des revendications 1 à 3 caractérisé en ce que l'adsorbant à base d'alumine est issu d'une mise en forme par coagulation en gouttes ou extrusion et présente une surface spécifique d'au moins 20 m²/g.

7. Procédé selon l'une des revendications 1 à 6 pour l'adsorption des composés organométalliques chélatés à base des métaux choisis parmi ceux des groupes IVB, VB, VIB, VIIB, VIII, IB, IIB.

8. Procédé selon l'une des revendications 1 à 7 pour l'adsorption des composés organométalliques chélatés à base de vanadium, de titane, de zirconium ou de cuivre.

9. Procédé selon l'une des revendications 1 à 8 pour l'adsorption des composés organométalliques chélatés à base des chélates choisis parmi l'acétylacétone, le 2-éthylhexanediol-1,3, le di-2-éthyl-hexylphosphate.

10. Procédé selon l'une des revendications 1 à 9 pour la purification des polyoléfines obtenues par polymérisation d'oléfines en présence d'un système de catalyseur de coordination.

## Patentansprüche

1. Verfahren zur Adsorption organometallischer Chelatverbindungen, dadurch gekennzeichnet, daß man diese Verbindungen mit einem Adsorptionsmittel auf der Basis von Aluminiumoxid kontaktiert, das aus einer Formgebung durch Tröpfchencoagulation oder durch Extrusion stammt oder durch Grobzerkleinerung erhalten wurde und über ein Volumen der Poren von einem Durchmesser größer als 80 Å von wenigstens 0,15 cm³/g und eine Granulometrie von weniger als 4 mm verfügt.

2. Verfahren nach Anspruch 1, dadurch gekennzeichnet, daß das Adsorptionsmittel wenigstens eine Verbindung eines Elements umfaßt, das aus der Gruppe, welche die Alkalis und die Erdalkalis umfaßt, gewählt ist.

3. Verfahren nach Anspruch 2, dadurch gekennzeichnet, daß der Gehalt der Verbindung des aus der Gruppe Alkalis und Erdalkalis gewählten Elementen zwischen 15 mmol und 750 mmol auf 100 g Aluminiumoxid resultiert.

4. Verfahren nach einem der Ansprüche 1 bis 3, dadurch gekennzeichnet, daß das Adsorptionsmittel auf der Basis von Aluminiumoxid erhalten wird durch Grobzerkleinerung und über eine spezifische Oberfläche von mehr als 200 m²/g verfügt.

5. Verfahren nach einem der Ansprüche 1 bis 4, dadurch gekennzeichnet, daß das Adsorptionsmittel auf der Basis von Aluminiumoxid durch Grobzerkleinerung von Aluminiumoxidkugeln erhalten wird.

6. Verfahren nach einem der Ansprüche 1 bis 3, dadurch gekennzeichnet, daß das Adsorptionsmittel auf der Basis von Aluminiumoxid aus einer Formgebung durch Tröpfchencoagulation oder Extrusion stammt und über eine spezifische Oberfläche von wenigstens 20 m²/g verfügt.

7. Verfahren nach einem der Ansprüche 1 bis 6 zur Adsorption der organometallischen Chelatverbindungen auf der Basis der Metalle, die gewählt sind aus solchen der Gruppen IVB, VB, VIB, VIIB, VIII, IB, IIB.

8. Verfahren nach einem der Ansprüche 1 bis 7 zur Adsorption der organometallischen Chelatverbindungen auf der Basis von Vanadium, Titan, Zirkonium oder Kupfer.

9. Verfahren nach einem der Ansprüche 1 bis 8 zur Adsorption der organometallischen Chelatverbindungen auf der Basis der Chelate, gewählt aus Acetylaceton, 2-Ethylhexandiol-1,3, sowie di-2-Ethyl-Hexylphosphat.

10. Verfahren nach einem der Ansprüche 1 bis 9 zur Reinigung der Polyolefin, die durch Polymerisation von Olefinen in Anwesenheit eines Koordinations-Katalysatorsystems erhalten wurden.

## Claims

1. Process for the adsorption of chelated organometallic compounds which comprises contacting said compounds with an alumina-based adsorbent, said adsorbent resulting from shaping by coagulation in droplets (oil drop) or by extrusion or being obtained by crushing, and said adsorbent having a volume of pores with a diameter greater than 80 Å of at least 0.15 cm³/g and a particle size of less than 4 mm.

2. Process according to claim 1 wherein the adsorbent comprises at least one alkali metal or alkaline earth metal compound.

3. Process according to claim 2 wherein the alkali metal or alkaline earth metal compound is present in an amount of from 15 mmol to 750 mmol per 100 g of alumina.

4. Process according to any one of claims 1 to 3 wherein the alumina-based adsorbent is obtained by crushing and has a specific surface area of greater than 200 m²/g.

5. Process according to any one of claims 1 to 4 wherein the alumina-based adsorbent is obtained by crushing alumina beads.

6. Process according to any one of claims 1 to 3 wherein the alumina-based adsorbent has resulted from shaping by coagulation in droplets (oil drop) or extrusion and has a specific surface area of at least 20 m²/g.

7. Process according to any one of claims 1 to 6 wherein the chelated organometallic compounds are based on metals chosen from groups IVB, VB, VIB, VIIB, VIII, IB and IIB of the Periodic Table.

8. Process according to claim 7 wherein the chelated organometallic compounds are based on vanadium, titanium, zirconium or copper.

9. Process according to any one of claims 1 to 8 wherein the chelated organometallic compounds are based on chelating agents chosen from acetylacetone, 2-ethylhexane-1,3-diol and di-2-ethylhexyl phosphate.

10. Process according to any one of claims 1 to 9 for the purification of polyolefins obtained by polymerization of olefins in the presence of a coordination catalyst system.
